# EUROPEAN PATENT SPECIFICATION

(11) **EP 1 055 401 B1**
(45) Date of publication and mention of the grant of the patent: **01.10.2003**
(21) Application number: 00111401.6
(22) Date of filing: 26.05.2000
(51) Int. Cl.: A61F 2/06, A61B 17/11

(54) **Device for anastomosis of blood vessels**
Vorrichtung zur Anastomose von Blutgefässen
Dispositif d'anastomose de vaisseaux sanguins

(30) Priority: 26.05.1999 JP 14727899; 16.07.1999 JP 20331899
(43) Date of publication of application: 29.11.2000
(60) Divisional application: 02023220.3
(73) Proprietor: NEC TOKIN Corporation, Sendai-shi, Miyagi (JP)
(72) Inventor: Sato, Akira, Sendai-shi, Miyagi (JP); Suzuki, Masao, c/o Tokin Corporation, Sendai-shi, Miyagi (JP); Furukawa, Akihisa, c/o Tokin Corporation, Sendai-shi, Miyagi (JP)
(74) Representative: Prüfer, Lutz H., Dipl.-Phys.

(56) References cited:
- WO-A-00/27313
- WO-A-99/55254
- US-A- 5 254 127
- US-A- 5 540 701
- US-A- 5 755 778

## Description

### Background of the Invention:

This invention relates to an anastomosis member for anastomosing blood vessels and an anastomosis method using the anastomosis member.

An anastomosis member serves to perform the anastomosis of two normally distinct hollow organs, such as blood vessels, to form a passage therethrough.

In a conventional surgical operation, the anastomosis of the blood vessels is generally carried out by suturing with a needle and a suture filament. When the blood vessels are anastomosed or joined to each other, a blood flow must temporarily be interrupted. If an increased number of sites are required to be anastomosed, the time of interrupting the blood flow is unfavorably extended.

In the surgical operation of a living body, it is required to use auxiliary means such as extracorporeal circulation or controlled hypothermia if it is presumed that the time of interrupting the blood flow exceeds an allowable time for the living body. It is difficult to suture the blood vessels under arteriosclerosis with the needle and the suture filament if those vessels have calcification. In case where the blood vessels are fragile, they must be reinforced to avoid the risk.

The technique of using a stent in the anastomosis of the blood vessels with heavy calcification is reported. In this technique, an artificial blood vessel is inserted into a host blood vessel to partially overlap each other. The stent is retained in the blood vessels at the overlapping portion to press-fit the blood vessels to each other. Thus, the anastomosis is carried out. For example, a type of the stent is disclosed in U.S. Patent No. 6,017,362.

In the technique of anastomosing the blood vessels by the use of the stent, the blood vessels are simply press-fitted by the elasticity of the stent and may be undesirably released from each other by, for example, the beat of the artery. Thus, the anastomosis by the use of the existing stent is insufficient in fixing or engaging force and in reliability.

US 5,254,127 discloses an anastomosis device according to the preamble of claim 1.

### Summary of the Invention:

It is therefore a technical object of this invention to provide an anastomosis device capable of safely and quickly carrying out the anastomosis of blood vessels.

It is another object of this invention to provide an anastomosis device improved in reliability by the use of a technique of applying an engaging force only to an adventitia of a blood vessel which is stronger than an intima of the blood vessel.

It is still another object of this invention to provide an anastomosis device capable of carrying out the anastomosis of blood vessels substantially equal in outer diameter and in inner diameter.

The object is solved by an anastomosis device according to claim 1, 4 or 5. Further developments are given in the dependent claims.

According to this invention, there is provided an anastomosis member to be arranged at an anastomosed site of first and second blood vessels to carry out the anastomosis of the first and the second blood vessels, the anastomosis member having a generally cylindrical body comprising at least one plate member to be brought into contact with both of the first and the second blood vessels, the plate member having a plurality of protrusions formed on at least one of opposite surfaces thereof to be engaged with at least one of the first and the second blood vessels so as to prevent the dislocation of the first and the second blood vessels at the anastomosed site.

Preferably, the anastomosis member has a plurality of the generally cylindrical bodies and at least one connecting member connecting the generally cylindrical bodies to one another.

Preferably, the anastomosis member has an elasticity so as to be compressed and expanded in diameter.

Preferably, the anastomosis member has a stress-strain characteristic including a plurality of different kinds of regions at least corresponding to a low-rigidity part deformable along the curvatures of the first and the second blood vessels to be tightly fitted thereto, and a spring region which is compressible and self-expandable in diameter.

Preferably, the generally cylindrical body comprises a plurality of the plate members connected to one another in a zigzag pattern.

Preferably, the generally cylindrical body comprises at least one plate member wound into a helical shape

Preferably, the generally cylindrical body comprises a plurality of the plate members connected to one another in a lattice pattern.

Preferably, the generally cylindrical body comprises a plurality of the plate members connected to one another in a rhombic pattern.

Preferably, the generally cylindrical body comprises a plurality of the plate members arranged in parallel to one another and a plurality of connecting members connecting the plate members to one another.

Preferably, each of the connecting members is an elastic wire member.

Preferably, the plate member is made of a stainless steel plate or a shape memory alloy selected from a TiNi alloy and a beta Ti alloy.

According to this invention, there is also provided an anastomosis device for use in the anastomosis of first and second blood vessels, the anastomosis device comprising a combination of an anastomosis member arranged on an outer surface of at least one of the first and the second blood vessels and a stent to be arranged in a lumen of the at least one of the first and the second blood vessels, the anastomosis member having a generally cylindrical body comprising at least one plate member to be brought into contact with both of the first and the second blood vessels, the plate member having a plurality of protrusions formed on at least one of opposite surfaces thereof to be engaged with at least one of the first and the second blood vessels.

Preferably, the stent Is made of a stainless steel plate or a shape memory alloy selected from a TiNi alloy and a beta Ti alloy.

Preferably, the anastomosis device further is suited for being engaged by an additional blood vessel.

### Brief Description of the Drawing:

Fig. 1 is a perspective view of an astomosis member according to a first embodiment of this invention;
Fig. 2 is a perspective view of an astomosis member according to a second embodiment of this invention;
Fig. 3 is a perspective view of an anastomosis member according to a third embodiment of this invention;
Fig. 4 is a perspective view of an anastomosis member according to a fourth embodiment of this invention;
Fig. 5 is a perspective view of an anastomosis member according to a fifth embodiment of this invention;
Fig. 6 is a perspective view of an anastomosis member according to a sixth embodiment of this invention;
Fig. 7 is a perspective view of an anastomosis member according to a seventh embodiment of this invention;
Fig. 8 is a perspective view of an anastomosis member according to an eighth embodiment of this invention;
Fig. 9 is a development view showing the eighth anastomosis member of in Fig. 8;
Fig. 10 is a transversal sectional view illustrating a first anastomosis example of blood vessels using the first anastomosis member of Fig. 1;
Fig. 11 is a transversal sectional view illustrating a second anastomosis example of blood vessels using the fifth anastomosis member of Fig. 5;
Fig. 12 is a transversal sectional view illustrating a third anastomosis example using a two-piece anastomosis device;
Fig. 13 is a transversal sectional view illustrating a fourth anastomosis example using a three-piece anastomosis device;
Fig. 14 is a deployed plan view of an anastomosis member according to a ninth embodiment of this invention;
Fig. 15 is a transversal sectional view illustrating a fifth anastomosis example using a composite anastomosis device; and
Fig. 16 is a cross-sectional view taken along in a line XVI-XVI in Fig. 15.

### Description of the Preferred Embodiments:

Now, description will be made about several preferred embodiments of this invention with reference to the drawing.

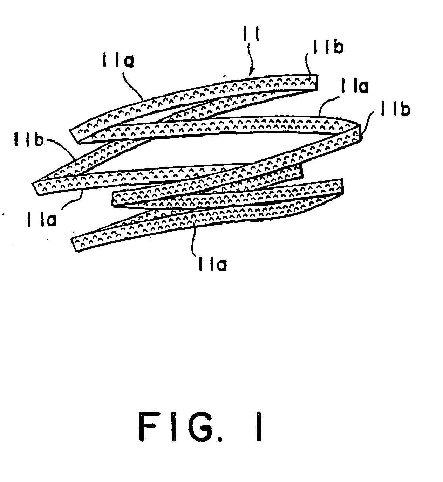

At first referring to Fig. 1, an anastomosis member 11 according to a first embodiment of this invention has a generally cylindrical body comprising a plurality of plate members or sections 11a connected to one another in a zigzag pattern extending along a cylindrical surface of an imaginary tube. Each of the plate members 11 a has opposite surfaces having a plurality of protrusions 11 b formed thereon.

The anastomosis member 11 is prepared in the following manner. At first, the plate members 11 a are formed. For example, each of the plate members 11a comprises a strip-like metal plate having a thickness of 0.2mm and a width of 2.0mm. Each of the protrusions 11 b has a height of 70µm and a diameter of 30 µm. The protrusions 11 b are arranged at a pitch of 0.3mm on the opposite surfaces of each plate member 11a. Thereafter, the plate members 11a are connected at their ends to one another by welding to extend in a zigzag pattern and to form the generally cylindrical body as the anastomosis member 11.

Alternatively, the anastomosis member 11 may be prepared from a sheet-like plate material. At first, the protrusions 11b are formed on the plate material. By the use of a laser or wire electric discharge, the plate material is cut into a zigzag pattern and then rolled into a cylindrical shape. Finally, rolled ends are welded to each other.

For example, the anastomosis member 11 thus prepared forms an imaginary tube having a cylindrical diameter of 8mm and an axial length of 10mm. The anastomosis member 11 is elastically variable in shape and in diameter.

The anastomosis member 11 can be produced in a different manner. Specifically, the plate member 11 a is made of a stainless steel material (SUS316) subjected to annealing and having a low rigidity. For example, the anastomosis member 11 has a cylindrical diameter of 6.5mm and an axial length of 10mm.

Alternatively, the anastomosis member 11 may be produced by bending a single long strip-like stainless steel plate in a zigzag pattern having a plurality of short strip-like plate sections 11a and then rounding the zigzag pattern of the stainless steel plate into a cylindrical shape.

In the first embodiment, the anastomosis member 11 has a zigzag pattern. However, the anastomosis member 11 may have various other patterns which will be described below.

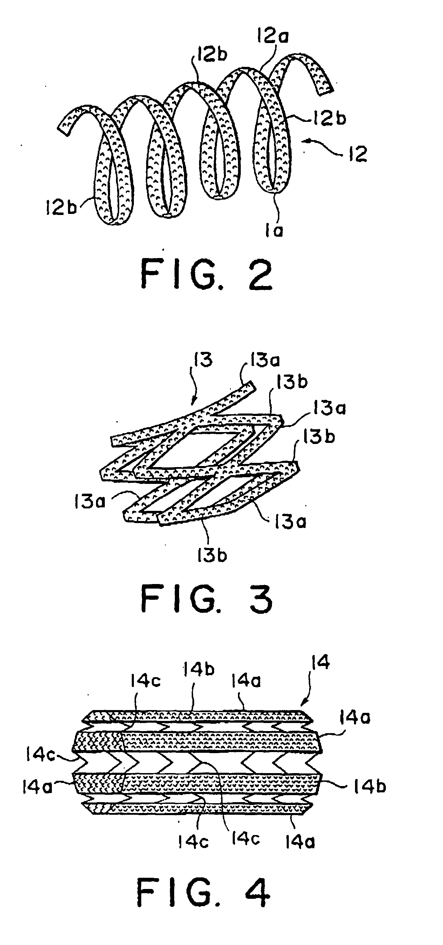

Referring to Fig. 2, an anastomosis member 12 according to a second embodiment of this invention has a generally cylindrical body comprising a helical coil formed by bending or winding a single long strip-like plate member 12a. Like in the first embodiment, the plate member 12a is provided with a plurality of protrusions 12b formed on opposite surfaces thereof. Instead of the single plate member 12a as the helical coil having a predetermined length, the anastomosis member 12 may comprise a plurality of helical coil elements which are connected in series to one another.

Referring to Fig. 3, an anastomosis member 13 according to a third embodiment of this invention has a generally cylindrical body comprising a plurality of strip-like plate members 13a connected to one another in a rhombic or a lattice pattern. Like in the foregoing embodiments, the plate member 13a is provided with a plurality of protrusions 13b formed on opposite surfaces thereof.

Referring to Fig. 4, an anastomosis member 14 according to a fourth embodiment of this invention has a generally cylindrical body comprising a plurality of strip-like plate members 14a equal in length and arranged in parallel to one another with an angular space kept from one another. These plate members 14a are connected by a plurality of wire connecting members 14c to form the generally cylindrical body as the anastomosis member 14. Like in the foregoing embodiments, the plate member 14a is provided with a plurality of protrusions 14b formed on its opposite surfaces.

For example, each of the plate members 14a comprises a stainless steel plate subjected to annealing and having a low rigidity. For example, each of the plate members 14a has an axial length of 10mm, a thickness of 0.2mm, and a width of 1.2mm. The anastomosis member 14 has a diameter of 8mm. Each of the connecting members 14c comprises a stainless steel wire (SUS304WP) having a length of 2mm and a diameter of 0.2mm. In this embodiment, the connecting members 14c comprise spring wires which are equal in length to each other and each of which is bent to form an angled portion having an acute angle.

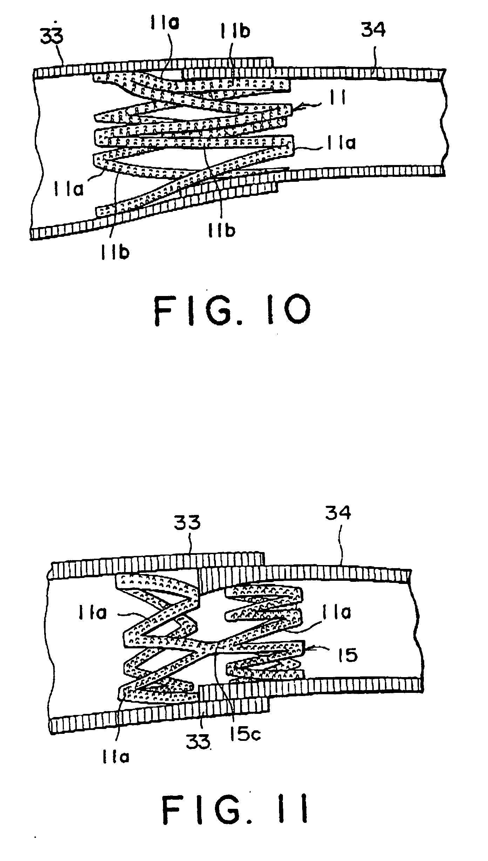

Specifically, the anastomosis member 14 has a low-rigidity part (plate members 14a) deformable along the curvatures of first and second blood vessels (Fig. 10, 33 and 34) to be tightly fitted thereto, and a self-expandable spring part (connecting members 14c). Thus, the anastomosis member 14 has a stress-strain characteristic including at least two different kinds of regions.

The protrusions 14b are formed on the opposite surfaces of each plate member 14a. The plate members 14a and the connecting members 14c are welded to each other to form the generally cylindrical body as the anastomosis member 14. The anastomosis member 14 can be expanded and compressed by changing the angles of the angled portions of the connecting members 14c.

As described above, the plate members 14a and the connecting members 14c are different from each other in stress-strain characteristic. The plate members 14a are soft and low in rigidity. Therefore, the anastomosis member 14 is readily deformable in conformity with the curvatures of the first and the second blood vessels without local pressure concentration in the first and the second blood vessels. Thus, the anastomosis member 14 can uniformly apply the pressure upon the first and the second blood vessels. Because of presence of the protrusions 14b, the anastomosis member 14 can be engaged with the first and the second blood vessels with a large frictional force.

It is noted here that the shape of the anastomosis member 14 is not restricted to that illustrated in Fig. 4. The connecting member 14c can be formed into a rhombic shape or any other appropriate shape as far as the connecting members 14c can hold the plate member 14a and are self-expandable. The plate member 14a is not restricted to the shape described in this embodiment but may have any other appropriate shape matching the configurations of the first and the second blood vessels.

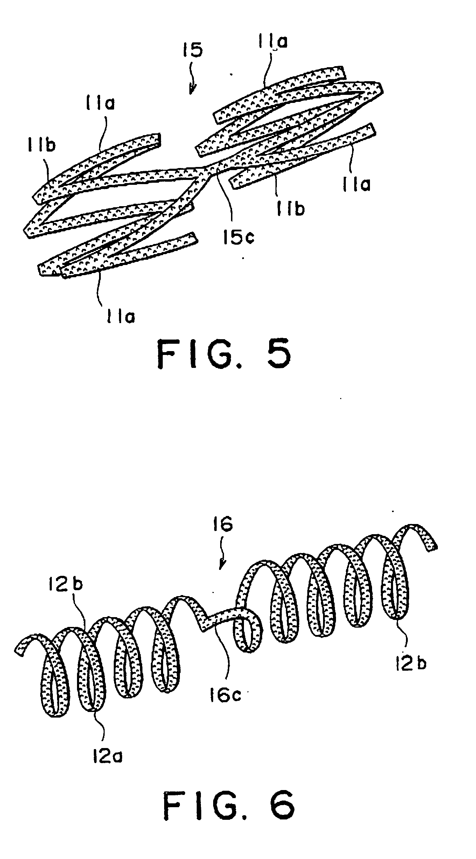

Referring to Fig. 5, an anastomosis member 15 according to a fifth embodiment of this invention comprises a pair of generally cylindrical bodies connected by a connecting portion 15c. Each of the generally cylindrical bodies is similar in structure to the generally cylindrical body of the anastomosis member 11 in Fig. 1. The connecting portion 15c is made of a material same as that of plate members 11a.

Referring to Fig. 6, an anastomosis member 16 according to a sixth embodiment of this invention comprises a pair of generally cylindrical bodies connected by a connecting portion 16c. Each of the generally cylindrical bodies is similar in structure to the generally cylindrical body of the anastomosis member 12 in Fig. 2. The connecting portion 16c is made of a material same as that of plate members 12a.

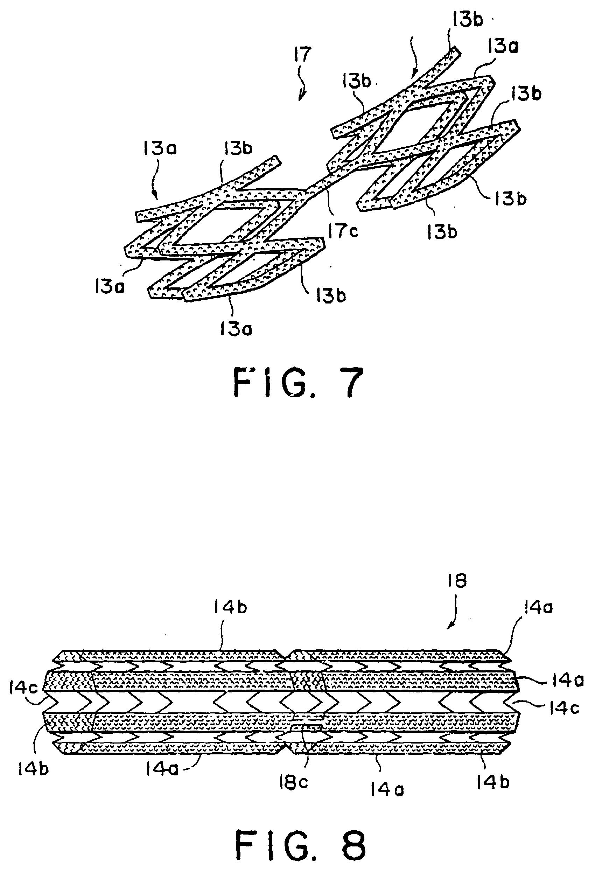

Referring to Fig. 7, an anastomosis member 17 according to a seventh embodiment of this invention comprises a pair of generally cylindrical bodies connected by a connecting portion 17c. Each of the generally cylindrical bodies is similar in structure to the generally cylindrical body of the anastomosis member 13 in Fig. 3. The connecting portion 17c is made of a material same as that of plate members 13a.

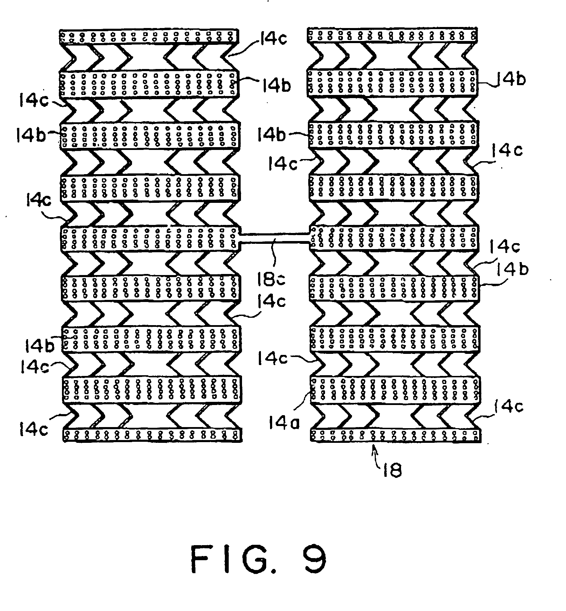

Referring to Figs. 8 and 9, an anastomosis member 18 according to an eighth embodiment of this invention comprises a pair of generally cylindrical bodies connected by a connecting portion 18c. Each of the generally cylindrical bodies is similar in structure to the generally cylindrical body of the anastomosis member 14 in Fig. 4. The connecting portion 18c is made of a material same as that of plate members 14a. The connecting portion 18c is smaller in width than each plate member 14a.

In the foregoing embodiments, the plate members 11a, 12a, 13a, and 14a are provided with the protrusions 11b, 12b, 13b, and 14b formed on both of the opposite surfaces thereof, respectively. Alternatively, the protrusions may be formed on only one of the opposite surfaces thereof. Each of the anastomosis members 11 through 18 is not restricted to the pattern described in each of the foregoing embodiments but may have any appropriate pattern as far as the diameter can flexibly be changed.

As a material of each of the plate members 11a, 12a, 13a, and 14a in the foregoing embodiments, use may be made of a stainless steel plate, a TiNi alloy and a TiNi-X alloy (X = Cr, V, Cu, Fe, Co, etc) having superelasticity at a living body temperature. Furthermore, use may also be made of a wide variety of shape memory alloys, such as a Cu-based alloy and a Fe-based alloy, as well as a beta Ti alloy. Taking the biocompatibility and the toxicity into consideration, the opposite surfaces of the plate members 11a, 12a, 13a, and 14a may be coated with titanium or the like.

Now, description will be made of several specific examples of the anastomosis of the first and the second blood vessels.

Referring to Fig. 10, a first example of the anastomosis will be described. Herein, the anastomosis is carried out by the use of the anastomosis member 11 illustrated in Fig. 1. In the following description, the similar parts are designated by like reference numerals. It will be noted here that the size of the anastomosis member 11 in this example is slightly different from that mentioned in conjunction with Fig. 1.

As illustrated in Fig. 10, the anastomosis member 11 is arranged over a host blood vessel 33 as the first blood vessel and an artificial blood vessel 34 as the second blood vessel. An end portion of the artificial blood vessel 34 is inserted into an end portion of the host blood vessel 33. The host and the artificial blood vessels 33 and 34 overlap each other to form an anastomosed site. The anastomosis member 11 is arranged in contact with intimae of the host and the artificial blood vessels 33 and 34.

Since the anastomosis member 11 comprises the plate members 11a connected in a zigzag pattern, the anastomosis member 11 can be brought into contact with the host and the artificial blood vessels 33 and 34 to expand the host and the artificial blood vessels 33 and 34 independently of each other. Because the protrusions 11 b provide a large frictional force, the anastomosis member 11 can be securely engaged with the intimae of the host and the artificial blood vessels 33 and 34. Therefore, even under the beat of the artery, the dislocation of the host and the artificial blood vessels 33 and 34 with respect to each other can be avoided by the use of the anastomosis member 11.

By a hand of a surgeon, the anastomosis member 11 is inserted into lumens of the host and the artificial blood vessels 33 and 34. For example, if the artificial blood vessel 34 has a diameter of 6mm, the anastomosis member 11 is compressed to a reduced diameter of 6mm or less and then inserted into the lumen of the artificial blood vessel 34. Furthermore, the anastomosis member 11 is inserted also into the lumen of the host blood vessel 33. The host and the artificial blood vessels 33 and 34 are made to approach each other to form an overlapping portion. At this time, the anastomosis member 11 is self-expanded from a compressed state. Therefore, the anastomosis can be quickly and easily carried out.

From the foregoing description, it will readily be understood that the anastomosis members 12, 13, and 14 illustrated in Figs. 2 through 4 may also be used instead of the anastomosis member 11. In this case also, the end portions of the host and the artificial blood vessels 33 and 34 can be expanded independently of each other so that the anastomosis is reliably carried out.

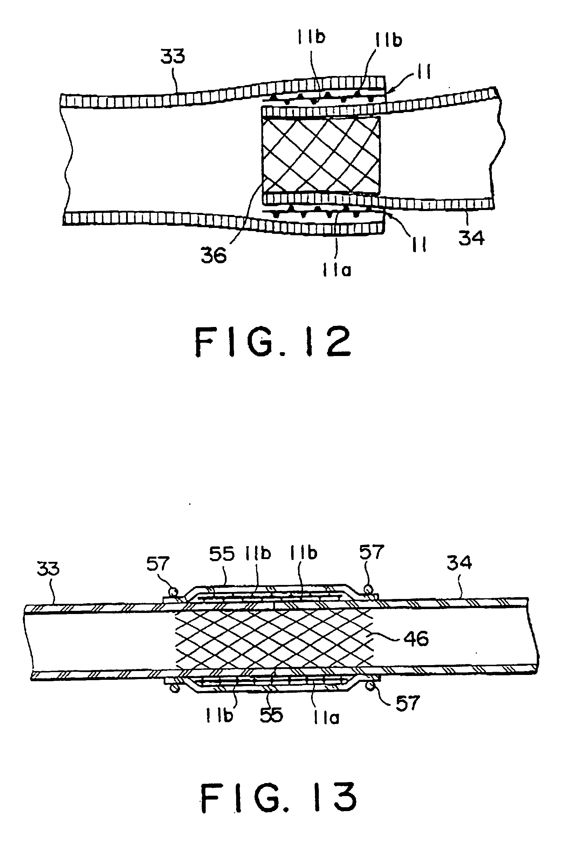

Referring to Fig. 11, a second example of the anastomosis will be described. Herein, the anastomosis is carried out by the use of the anastomosis member 15 illustrated in Fig. 5. In the following description, similar parts are designated by like reference numerals. It will be noted here that the size of the anastomosis member 15 in this example is slightly different from that mentioned in conjunction with Fig. 5.

As illustrated in Fig. 11, the anastomosis member 15 is attached to the host blood vessel 33 and the artificial blood vessel 34. The anastomosis member 15 is arranged over the lumens of the host and the artificial blood vessels 33 and 34 to be anastomosed. The end portion of the artificial blood vessel 34 is inserted into the end portion of the host blood vessel 33.

When the anastomosis member 15 is arranged in the host and the artificial blood vessels 33 and 34, the anastomosis member 15 can expand the end portions of the host and the artificial blood vessels 33 and 34 independently of each other. Thus, the anastomosis is reliably carried out.

From the foregoing description, it will readily be understood that the anastomosis members 16,17, and 18 illustrated in Figs. 6 through 8 may also be used instead of the anastomosis member 15. In this case also, the end portions of the host and the artificial blood vessels 33 and 34 can be engaged by the anastomosis member 15 to be expandable independently of each other. Thus, the anastomosis is reliably carried out.

Referring to Fig. 12, a third example of the anastomosis will be described. Herein, the anastomosis is carried out by the use of a two-piece anastomosis device comprising a combination of the anastomosis member 11 in Fig. 1 and a woven tubular stent 36. In the following description, similar parts are designated by like reference numerals. It will be noted here that the size of the anastomosis member 11 in this example is slightly different from that mentioned in conjunction with Fig. 1.

As illustrated in Fig. 12, the anastomosis member 11 is arranged in an anastomosed site between the host blood vessel 33 and the artificial blood vessel 34. The end portion of the artificial blood vessel 34 is inserted into the end portion of the host blood vessel 33. At the anastomosed site, the end portions of the host and the artificial blood vessels 33 and 34 overlap each other with the anastomosis member 11 interposed therebetween. In the lumen of the artificial blood vessel 34 at the anastomosed site, the stent 36 is arranged.

For example, the stent 36 comprises a shape memory alloy such as a TiNi alloy having a cylindrical shape and a lattice pattern. For example, the stent 36 is designed to have a cylindrical shape with a final expanding diameter of 7mm. The stent 36 has an axial length of 10mm. The stent 36 has superelasticity at and around the body temperature of the living body.

As illustrated in Fig. 12, the stent 36 expands the artificial blood vessel 34 outwards in a radial direction. The stent 36 is inserted into the lumen of the artificial blood vessel 34 having an outer diameter of 6mm and an inner diameter of 5mm. Then, the artificial blood vessel 34 is inserted into the host blood vessel 33 with the anastomosis member 11 interposed between the host and the artificial blood vessels 33 and 34.

The host and the artificial blood vessels 33 and 34 and the anastomosis member 11 are press-fitted by the stent 36. At this time, the protrusions 11b are engaged with the intima of the host blood vessel 33 and the adventitia of the artificial blood vessel 34. Thus, the host and the artificial blood vessels 33 and 34 are prevented from being dislocated even under the beat of the artery. Thus, the anastomosis is reliably carried out.

The stainless steel plate of the anastomosis member 11 is not restricted to SUS316 but may be any other appropriate product having a low rigidity and a flexibility. The material and the shape of the stent 36 are not restricted to those given in this embodiment but may be appropriately selected taking into account the sizes of the host blood vessel 33 and the artificial blood vessel 34 as well as an expanding force of the stent 36.

Referring to Fig. 13, a fourth example of the anastomosis will be described. Herein, the anastomosis is carried out by the use of a three-piece anastomosis device comprising a combination of the anastomosis member 11 in Fig. 1, a woven tubular stent 46, and an additional blood vessel 55. In this example also, the anastomosis member 11 serves as a securing member for securing the two blood vessels in the anastomosed state. In the following description, similar parts are designated by like reference numerals. It will be noted here that the size of the anastomosis member 11 in this example is slightly different from that mentioned in conjunction with Fig. 1.

The anastomosis member 11 is attached to the host and the artificial blood vessels 33 and 34. An end face of the artificial blood vessel 34 is abutted to an end face of the host blood vessel 33. The anastomosis member 11 is arranged on outer surfaces of the host and the artificial blood vessels 33 and 34 to extend over both of the host and the artificial blood vessels 33 and 34. The stent 46 is arranged in the lumens of the host and the artificial blood vessels 33 and 34 at an area corresponding to the anastomosis member 11.

Furthermore, the additional blood vessel 55 is arranged around the host and the artificial blood vessels 33 and 34 to cover the anastomosis member 11. The anastomosis member 11 and the additional blood vessel 55 are engaged with each other by the protrusions 11 b. The opposite ends of the additional blood vessel 55 are fastened to the host and the artificial blood vessels 33 and 34 by a filament 57 or a strap as a fastening member. The protrusions 11b may be formed only on one surface of the anastomosis member 11 to be engaged with the host and the artificial blood vessels 33 and 34.

More specifically, the anastomosis member 11 comprises a strip-like plate member 11a made of a stainless steel plate (SUS316) subjected to annealing and having a low rigidity. The plate member 11a has a thickness of 0.25mm. Each protrusion 11b has a height between 65 and 75µm and a diameter of 30µm. The protrusions 11 b are formed at a pitch of 0.2mm.

In this example of the anastomosis, the anastomosis member 11 has an inner diameter of 8mm, an axial length of 10mm, and a width of 0.4mm. The anastomosis member 11 is flexibly variable in shape and in cylindrical diameter.

The stent 46 is made of a material similar to that of the stent 36 illustrated in Fig. 12 and has a lattice pattern. For example, the stent 46 is designed to have a cylindrical shape with a final expanding diameter of 7mm and an axial length of 20mm.

In the anastomosis, one axial half of the stent 46 is inserted into the lumen of the artificial blood vessel 34, for example, having an outer diameter of 8mm and an inner diameter of 6mm. Then, the host and the artificial blood vessels 33 and 34 are abutted to each other. Simultaneously, the other axial half of the stent 46 is inserted into the lumen of the host blood vessel 33. The anastomosis member 11 preliminarily arranged on the outside of the artificial blood vessel 34 is positioned to extend over the outer surfaces of both of the host and the artificial blood vessels 33 and 34. Likewise, the additional blood vessel 55 arranged on the outside of the artificial blood vessel 34 is positioned around the anastomosis member 11. The additional blood vessel 55 has an inner diameter of 8.1 mm.

The opposite ends of the additional blood vessel 55 are fastened by the filament 57 as the fastening member to the outer surfaces of the host and the artificial blood vessels 33 and 34. The host and the artificial blood vessels 33 and 34 and the anastomosis member 11 are press-fitted by an internal pressure applied by the stent 46 and an external pressure applied by the additional blood vessel 55 fastened by the filament 57. At this time, the protrusions 11 b are engaged with the host and the artificial blood vessels 33 and 34. Thus, the host and the artificial blood vessels 33 and 34 are prevented by the anastomosis member 11 from being dislocated with respect to each other even under the beat of the artery. Therefore, the anastomosis is reliably carried out. Since the additional blood vessel 55 and the filament 57 are fastened to each other, blood leakage from the anastomosed site to the outside is prevented.

The anastomosis in the above-mentioned example can be manually and quickly carried out by a surgeon so that the time of interrupting the blood flow can be shortened.

In this example of the anastomosis, the engaging force of the protrusions 11b acts only on the adventitia of each blood vessel which is relatively strong as compared with the intima. Thus, the load upon the host and the artificial blood vessels 33 and 34 is small as compared with the case where the inner surfaces are used in engagement. Furthermore, the host and the artificial blood vessels 33 and 34 need not overlap each other so that the anastomosis is possible even if the host and the artificial blood vessels 33 and 34 are substantially equal in diameter to each other. The anastomosis member 11 can relatively flexibly cope with the difference in size between the host and the artificial blood vessels 33 and 34.

The anastomosis member 11 is not restricted to the pattern described above but may have any pattern as far as the cylindrical diameter is flexibly variable. The material of the anastomosis member 11 is not restricted to the stainless steel plate but may be any appropriate material having a low rigidity and a flexibility.

The stent 46 is not restricted to the shape described above but may have any shape as far as an appropriate expanding force is provided so as to engage the host and the artificial blood vessels 33 and 34 and the anastomosis member 11 with one another. For example, use may be made of various kinds of stents typically used for expanding constricted portions of blood vessels.

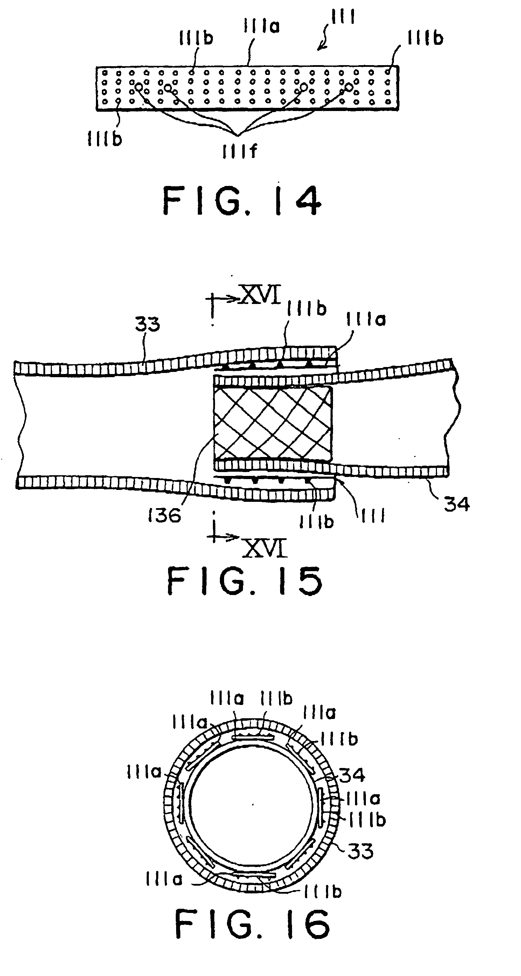

Referring to Fig. 14, an anastomosis member 111 according to a ninth embodiment of this invention comprises a plurality of plate members 111a although only one is illustrated in the figure. Each plate member 111a is provided with a plurality of protrusions 111b formed on only one of its opposite surfaces. The plate member 111a is substantially similar to the plate member 11a illustrated in Fig. 1 except that a plurality of through holes 111f are formed along its center line parallel to a longitudinal direction thereof. The through holes 111f allow a suture filament (not shown) to pass therethrough. The plate members 111a are sutured to the artificial blood vessel 34 by the suture filament passing through the through holes 111f.

Referring to Figs. 15 and 16, a fifth example of the anastomosis will be described. Herein, the anastomosis is carried out by the use of a composite anastomosis device comprising a combination of the anastomosis member 111 in Fig. 14 and a woven tubular stent 136. The stent 136 is substantially similar to the stent 36 illustrated in Fig. 12.

The plate members 111a are sutured to the artificial blood vessel 34 with their surfaces having the protrusions 111b faced outward. In the example being illustrated in Fig. 16, eight plate members 111a are arranged on the outer surface of the artificial blood vessel 34 to form an octagonal shape around the center axis of the artificial blood vessel 34.

As illustrated in Fig. 15, the stent 136 is inserted into the lumen of the artificial blood vessel 34. The end portion of the artificial blood vessel 34 is inserted into the end portion of the host blood vessel 33. At this time, the plate members 111a are arranged between the blood vessels 33 and 34 overlapping each other. The protrusions 111b formed on the one surface of the plate members 111a sutured to the artificial blood vessel 34 are faced to the inner surface of the host blood vessel 33. The host blood vessel 33 and the plate members 111a are press-fitted by a pressing force of the stent 136 and the host blood vessel 33 is engaged by the protrusions 111b. Thus, the host and the artificial blood vessels 33 and 34 are prevented from being dislocated with respect to each other. Thus, the anastomosis is reliably carried out.

The shape and the number of the plate members 111a are not restricted to those given in this embodiment but may be appropriately selected so that the host and the artificial blood vessels 33 and 34 are reliably engaged by the protrusions 111b.

In the foregoing, description is directed to the anastomosis of the host blood vessel 33 and the artificial blood vessel 34 inserted into the host blood vessel 33. However, this invention is also applicable to the case where the host blood vessel 33 is inserted into the artificial blood vessel 34 in dependence upon the condition of the host blood vessel 33. It will readily be understood that this invention is also applicable to the case where the first and the second blood vessels are both living blood vessels.

As described above, according to this invention, the anastomosis member having protrusions are arranged at the anastomosed site of the first and the second blood vessels so that the engaging force for engaging the first and the second blood vessels can be increased. The anastomosis member can achieve the safe anastomosis not only in a normal blood vessel but also in a diseased blood vessel. Furthermore, the anastomosis of the first and the second blood vessels can be quickly carried out.

The anastomosis member of this invention is highly reliable because the protrusions can be formed so that the engaging force acts only on the adventitia of each blood vessel which is stronger than the intima.

The anastomosis member can be attached to the first and the second blood vessels which are abutted at their end faces to each other with the stent inserted into the lumens of the first and the second blood vessels. Therefore, it is possible to carry out the anastomosis for the first and the second blood vessels substantially equal in diameter. In addition, the anastomosis member can flexibly cope with a slight difference in size between the first and the second blood vessels.

The anastomosis member can be arranged on the outside of the first and the second blood vessels. Furthermore, the additional blood vessel can be arranged on the outside of the anastomosis member and fastened by a filament or a strap in at least one position. With this structure, it is possible to exert a greater engaging force and to assure a sufficient fixing force. By the use of the additional blood vessel, it is possible to effectively prevent the blood leakage out of the anastomosed site.

The anastomosis member can be compressed and expanded by changing the angles of the angled portions of the connecting members. The plate member has a stress-strain characteristic different from that of the connecting member and is flexible and low in rigidity.

Finally, the anastomosis member is readily deformable in correspondence to the curvatures of the first and the second blood vessels. Therefore, it is possible to uniformly apply the pressure to the first and the second blood vessels without causing local concentration of the pressure.

## Claims

1. An anastomosis device for use in the anastomosis of first and second blood vessels (33, 34), said anastomosis device comprising an anastomosis member having a generally cylindrical body comprising at least one plate member (11a, 111a) to be brought into contact with both of said first and said second blood vessels, said plate member having a plurality of protrusions (11b, 111b) formed on at least one of opposite surfaces thereof to be engaged with at least one of said first and said second blood vessels, **characterized in that** said anastomosis device further comprises a combination of said anastomosis member (11, 111) arranged on an outer surface of at least one of said first and said second blood vessels and a stent (36, 46, 136) to be arranged in a lumen of said at least one of the first and the second blood vessels to prevent the dislocation of the first and the second blood vessels at the anastomosed site.

2. An anastomosis device as claimed in claim 1, wherein said stent (36, 46, 136) is made of a stainless steel plate or a shape memory alloy selected from a TiNi alloy and a beta Ti alloy.

3. An anastomosis device as claimed in claim 1 or 2, suitable for being engaged on the outer surface by an additional blood vessel (34, 55).

4. An anastomosis device as claimed in claim 1 **characterized in that** said anastomosis member comprising a plate member (11a to 14a, 111a) with a plurality of protrusions (11b to 14b, 111b) formed on at least one of opposite surfaces thereof, wherein said anastomosis member - stent combination is designed such that said stent is capable of being inserted into an end portion of a lumen of one of said first and said second blood vessels;
that said anastomosis member is capable of being inserted to be interposed between said first and said second blood vessels at an anastomosed site where said first and said second blood vessels overlap each other; wherein said protrusions can be brought into contact with said first and said second blood vessels;
wherein said stent is capable of being expanded outwards in a radial direction such that said first and said second blood vessels and said anastomosis member are press-fitted by the stent at said anastomosed site; thereby preventing the dislocation of said first and said second vessels at said anastomosed site.

5. An anastomosis device as claimed in claim 1 **characterized in that** said anastomosis member comprising a plate member (11a to 14a, 111a) with a plurality of protrusions (11b to 14b, 111b) formed on at least one of opposite surfaces thereof, wherein said anastomosis member - stent combination is designed such that said stent is capable of being inserted to extend over lumens of said first and said second blood vessels;
that said anastomosis member is capable of being arranged around outer surface of and to extend over both said first and said second blood vessels; wherein
said protrusions can be brought into contact with an adventitia of each of said first and said second blood vessels;
thereby preventing the dislocation of said first and said second blood vessels at said anastomosed site.

6. An anastomosis device as claimed in claim 4 or 5, wherein each of said plate members (111a) has a plurality of through holes (111f) in order to allow a fastening member to pass there through, said protrusions (111b) being formed only on one surface of said plate member.

7. An anastomosis device as claimed in one of claims 4 to 6, wherein said anastomosis member - stent combination further, is suited for an additional blood vessel (55) to be placed on the outside of said anastomosis member (11) which is to be arranged around the outer surfaces of said first and said second blood vessels (33, 34); and
a fastening member (57) capable of fastening said additional blood vessel preferably in at least one position, said fastening member preferably being a filament or a strap.

## Patentansprüche

1. Anastomosevorrichtung zur Verwendung bei der Anastomose von ersten und zweiten Blutgefäßen (33, 34), wobei die Anastomosevorrichtung ein Anastomoseelement umfasst, welches einen im allgemeinen zylindrischen Körper aufweist, der zumindest ein Plattenelement (11a, 111a) umfasst, welches sowohl mit dem ersten als auch dem zweiten Blutgefäß in Kontakt zu bringen ist, wobei das Plattenelement eine Vielzahl von Vorsprüngen (11b, 111b) aufweist, die auf mindestens einer der gegenüberliegenden Oberflächen davon gebildet sind zum Eingriff mit mindestens einem der ersten und zweiten Blutgefäße, **dadurch gekennzeichnet, dass** die Anastomosevorrichtung ferner eine Kombination des besagten Anastomoseelements (11, 111), welches bei einer äußeren Oberfläche von mindestens einem der ersten und zweiten Blutgefäße angeordnet ist, und eines Stents (36, 46, 136), welches in einem Lumen von mindestens einem der ersten und zweiten Blutgefäße anzuordnen ist, umfasst, um das Versetzen der ersten und zweiten Blutgefäße bei der anastomisierten Stelle zu verhindern.

2. Anastomosevorrichtung wie im Anspruch 1 beansprucht, wobei der Stent (36, 46, 136) aus einer rostfreien Stahlplatte oder einer Formerinnerungslegierung gefertigt ist, die aus einer TiNi-Legierung und einer β-Ti-Legierung ausgewählt ist.

3. Anastomosevorrichtung wie im Anspruch 1 oder 2 beansprucht, geeignet zum Eingriff bei der äußeren Oberfläche durch ein zusätzliches Blutgefäß (34, 55).

4. Anastomosevorrichtung wie im Anspruch 1 beansprucht, **dadurch gekennzeichnet, dass** das Anastomoseelement ein Plattenelement (11a-14a, 111a) mit einer Vielzahl von Vorsprüngen (11b-14b, 111b), die auf zumindest einer der gegenüberliegenden Oberflächen davon gebildet sind, umfasst, wobei die Anastomoseelement-Stent-Kombination so ausgestaltet ist, dass der Stent in der Lage ist, in einen Endabschnitt eines Lumens von einem der ersten und zweiten Blutgefäße insertiert zu werden;
dass das Anastomoseelement in der Lage ist, zwischen das erste und das zweite Blutgefäß bei der anastomisierten Stelle insertiert zu werden, wo die ersten und zweiten Blutgefäße einander überlappen;
wobei die Vorsprünge mit den ersten und zweiten Blutgefäßen in Kontakt gebracht werden können;
wobei der Stent befähigt ist, nach außen in einer radialen Richtung derart aufgeweitet zu werden, dass das erste und das zweite Blutgefäß sowie das Anastomoseelement bei der anastomisierten Stelle durch den Stent press-eingepasst sind; wodurch das Versetzen der ersten und zweiten Blutgefäße bei der anastomisierten Stelle verhindert ist.

5. Anastomosevorrichtung wie im Anspruch 1 beansprucht, **dadurch gekennzeichnet, dass** das Anastomoseelement ein Plattenelement (11a-14a, 111a) mit einer Vielzahl von Vorsprüngen (11b-14b, 111b), die zumindest bei einem der gegenüberliegenden Oberflächen davon gebildet sind, umfasst, wobei die Anastomoseelement-Stent-Kombination so ausgestaltet ist, dass der besagte Stent in der Lage ist, insertiert zu werden, dass er sich über die Lumen der ersten und zweiten Blutgefäße erstreckt;
dass das Anastomoseelement in der Lage ist, um die Außenoberflächen herum sowie über die ersten und zweiten Blutgefäße erstreckend angeordnet zu werden;
wobei die Vorsprünge mit einer Adventitia von jedem der ersten und zweiten Blutgefäße in Kontakt gebracht werden können;
wodurch das Versetzen der ersten und zweiten Blutgefäße bei der anastomisierten Stelle verhindert ist.

6. Anastomosevorrichtung wie im Anspruch 4 oder 5 beansprucht, wobei jedes der Plattenelemente (111a) eine Vielzahl von Durchgangslöchern (111f) aufweist, um ein Befestigungselement dort hindurch treten zu lassen, wobei die Vorsprünge (111b) nur auf einer Oberfläche des Plattenelements gebildet sind.

7. Anastomosevorrichtung wie in einem der Ansprüche 4 bis 6 beansprucht, wobei die Anastomoseelement-Stent-Kombination ferner dazu geeignet ist, dass ein zusätzliches Blutgefäß (55) auf die Außenseite des Anastomoseelements (11) zu platzieren ist, welches um die Außenoberflächen der ersten und zweiten Blutgefäße (33, 34) herum anzuordnen ist; und dass ein Befestigungselement (57) in der Lage ist, das zusätzliche Blutgefäß vorzugsweise bei zumindest einer Position zu befestigen, wobei das Befestigungselement vorzugsweise ein Faden oder ein Band ist.

## Revendications

1. Dispositif d'anastomose destiné à être utilisé dans l'anastomose d'un premier vaisseau sanguin et d'un second vaisseau sanguin (33,34), ce dispositif d'anastomose comprenant un élément d'anastomose muni d'un corps généralemcnt cylindrique comportant au moins un élément de plaque (11a, 111a) devant être amené en contact à la fois avec le premier vaisseau sanguin et avec le second vaisseau sanguin, cet élément de plaque comportant un certain nombre de saillies (11b, 111b) formées sur l'une au moins de ses surfaces opposées pour se raccorder avec l'un au moins des premier et second vaisseaux sanguins,
**caractérisé en ce que**
ce dispositif d'anastomose comprend en outre une combinaison de l'élément d'anastomose (11, 111) disposé sur une surface de l'un au moins des premier et second vaisseaux sanguins, avec un tubage (36, 46, 136) devant être disposé dans une lumière de l'un au moins des premier et second vaisseaux sanguins pour éviter le déboîtement de ces premier et second vaisseaux sanguins à l'endroit du site anastomosé.

2. Dispositif d'anastomose selon la revendication 1,
dans lequel
le tubage (36, 46, 136) est réalisé dans une plaque en acier inoxydable ou en alliage à mémoire de forme sélectionné parmi un alliage de TiNi et un alliage de Ti beta.

3. Dispositif d'anastomose selon la revendication 1 ou 2,
convenable pour s'engager sur la surface extérieure d'un vaisseau sanguin supplémentaire (34, 35).

4. Dispositif d'anastomose selon la revendication 1,
**caractérisé en ce que**
- l'élément d'anastomose comprend un élément de plaque (11a à 14a, 111a) muni d'un certain nombre de saillies (11b à 14b, 111b) formées sur l'une au moins de ses surfaces opposées, la combinaison de cet élément d'anastomose et du tubage étant conçue de façon que le tubage soit capable d'être introduit dans une partie d'extrémité d'une lumière de l'un des premier et second vaisseaux sanguins ;
- l'élément d'anastomose est capable d'être introduit pour s'interposer entre les premiers et second vaisseaux sanguins dans un site anastomosé où les premier el second vaisseaux sanguins se recouvrent. l'un l'autre ;
- les saillies peuvent être amenées en contact avec les premiers et second vaisseaux sanguins ; et
le tubage est capable d'être dilaté vers l'extérieur dans la direction radiale de façon que les premier et second vaisseaux sanguins ainsi que l'élément d'anastomose soient emboîtés à la presse au moyen du tubage à l'endroit du site anastomosé ;
de manière à éviter le déboîtement des premier et second vaisseaux sanguins à l'endroit du site anastomosé.

5. Dispositif d'anastomose selon la revendication 1,
**caractérisé en ce que**
- l'élément d'anastomose comprend un élément de plaque (11a à 14a, 111a) munie d'un certain nombre de saillies (11b à 14b, 111b) formées sur l'une au moins de ses surfaces opposées ;
- la combinaison de cet élément d'anastomose et du tubage étant conçue de façon que le tubage soit capable d'être introduit pour s'étendre sur les lumières des premier et second vaisseaux sanguins ;
- l'élément d'anastomose est capable d'être disposé autour des surfaces extérieures à la fois du premier vaisseau sanguin et du second vaisseau sanguin, et de s'étendre sur ces deux vaisseaux ; et
- les saillies peuvent être amenées en contact avec un élément fortuit de chacun des premier et second vaisseaux sanguins ;
de manière à éviter le déboitement des premier et second vaisseaux sanguins à l'endroit du site anastomosé.

6. Dispositif d'anastomose selon la revendication 4 ou 5,
dans lequel
chacun des éléments de plaque (111a) est percé d'un certain nombre de trous traversant (111f) pour permettre à des éléments de fixation de passer à travers ceux-ci, les saillies (111b) n'étant formées que sur une seule surface de l'élément de plaque.

7. Dispositif d'anastomose selon l'une quelconque des revendications 4 à 6
dans lequel
- la combinaison de l'élément d'anastomose et du tubage cst en outre conçue pour un vaisseau sanguin supplémentaire (55) devant être placé sur l'extérieur de l'élément d'anastomose (11) devant lui-même être déposé autour des surfaces extéricures des premier et second vaisseaux sanguins (33, 34); et
- un élément de fixation (57) est capable de fixer le vaisseau sanguin supplémentaire, de préférence dans au moins une position, cet élément de fixation étant de préférence un filament ou une bande.
